# EUROPEAN PATENT SPECIFICATION

(11) **EP 2 797 661 B1**
(45) Date of publication and mention of the grant of the patent: **24.01.2018**
(21) Application number: 12863623.0
(22) Date of filing: 16.12.2012
(51) Int. Cl.: A61N 1/06, A61B 18/12, A61B 18/14, A61B 18/00

(54) **SKIN TREATMENT DEVICE**
HAUTBEHANDLUNGSVORRICHTUNG
DISPOSITIF DE TRAITEMENT DE LA PEAU

(30) Priority: 28.12.2011 US 201161580687 P
(43) Date of publication of application: 05.11.2014
(73) Proprietor: Derma Dream Group Ltd., Tortola (VG)
(72) Inventor: Luzon, Josef, 405091 Bet Yehoshua (IL); Gurovich, Martin, 69350 Tel Aviv (IL)
(74) Representative: Stellbrink & Partner Patentanwälte mbB
(86) International application number: PCT/IL2012/050529
(87) International publication number: WO 2013/098815

(56) References cited:
- WO-A1-91/16859
- WO-A2-2011/058565
- JP-A- 2011 194 176
- JP-A- 2011 194 176
- US-A1- 2002 128 641
- US-A1- 2002 128 641
- US-A1- 2006 036 300
- US-A1- 2007 179 490
- US-A1- 2008 183 251
- US-A1- 2008 183 251
- US-A1- 2010 210 993
- US-A1- 2010 228 243
- US-B2- 6 662 054

## Description

### FIELD OF THE INVENTION

The present invention relates to a skin treatment device.

### BACKGROUND OF THE INVENTION

One goal of various beauty treatments is tightening of the skin so as to give the skin a smooth and non-wrinkled appearance. Various treatments for tightening the skin have been developed and applied over the centuries.

Massaging the skin has been considered to lead to skin tightening. Application of massage is claimed by proponents to benefit the skin by stimulating the vascular system, as well as by promoting the drainage of lymph from skin tissue. In addition to manual massage, various mechanical devices have been developed to massage the skin in a consistent and repeatable manner.

Other treatments for the skin are based on selective electro-thermolysis. In selective electro-thermolysis, layers of the skin are subjected to heating. In particular, delivery of radiofrequency (RF) electromagnetic energy to layers of the skin has been promoted as an effective way of heating the skin in a controlled manner for treatment purposes. Application of RF energy as a skin treatment has been described as increasing cell metabolism rates, increasing blood flow to the skin, causing adipocyte necrosis (shrinking of fat cells), and as stimulating remodeling of collagen.

US 2008/183251 describes devices for delivering RF electromagnetic energy to the skin. The devices include one or more electromagnetic RF generating units, multiple RF electrode groups and a controller for controllably applying RF energy to the skin through any selected RF electrode group or any selected RF electrode group combination selected from the multiple groups. The electrodes may be stationary or movable. The alternation of energy application through different electrode groups at different times, or the changing of the inter-electrode distance and configuration by using movable RF electrodes may reduce or prevent electrode overheating, control RF energy distribution within the skin and enable use of the devices for different skin treating applications.

It is an object of embodiments of the present invention to provide a device for effective treatment of the skin.

Other aims and advantages of embodiments of the present invention will become apparent after reading the present invention and reviewing the accompanying drawings.

### SUMMARY OF THE INVENTION

The invention is defined by the appended claims. There is provided, in accordance with some embodiments of the present invention, a device for treating skin, the device including

two mechanical fingers for placement in contact with the skin, at least one of the fingers being moveable so as to change a separation distance between the fingers and to gently pinch and release a region of skin between the mechanical fingers, the fingers being configured to concurrently conduct a generated radiofrequency electromagnetic signal to the skin so as to cause an electrical current to flow through the skin at a depth that corresponds to the separation distance; and

a temperature sensor embedded within, or in thermal contact with, a mechanical finger of said two mechanical fingers.

Furthermore, in accordance with some embodiments of the present invention, the device is shaped so as to be held by a single hand.

Furthermore, in accordance with some embodiments of the present invention, the device includes a motor and transmission for moving said at least one of the fingers.

Furthermore, in accordance with some embodiments of the present invention, the transmission includes a cam that is rotatable by the motor for changing a distance between the fingers.

Furthermore, in accordance with some embodiments of the present invention, the cam is located so as to contact the arm at a point between the distal end of the arm and the axis of the arm.

Furthermore, in accordance with some embodiments of the present invention, the device includes a spring for exerting an inward torque on at least one of the arms.

Furthermore, in accordance with some embodiments of the present invention, the device includes a controller for controlling operation of the motor.

Furthermore, in accordance with some embodiments of the present invention, the temperature sensor is embedded in one of the fingers.

Furthermore, in accordance with some embodiments of the present invention, the radiofrequency electromagnetic signal is generated in accordance with a skin temperature that is sensed by the temperature sensor.

Furthermore, in accordance with some embodiments of the present invention, the device is configured to interrupt generation of the radiofrequency electromagnetic signal if the sensed temperature exceeds a threshold temperature.

Furthermore, in accordance with some embodiments of the present invention, the device includes a control for operation by a user.

Furthermore, in accordance with some embodiments of the present invention, the device includes an indicator for indicating a status of the device.

Furthermore, in accordance with some embodiments of the present invention, the device includes a power supply.

Furthermore, in accordance with some embodiments of the present invention, the power supply includes a rechargeable battery or a replaceable battery.

### BRIEF DESCRIPTION OF THE DRAWINGS

In order to better understand the present invention, and appreciate its practical applications, the following Figures are provided and referenced hereafter. It should be noted that the Figures are given as examples only and in no way limit the scope of the invention. Like components are denoted by like reference numerals.
Fig. 1 shows a skin treatment device in accordance with an embodiment of the present invention;
Fig. 2 shows internal components of the skin treatment device shown in Fig. 1;
Fig. 3 is a block diagram of an electrical system of operation of a skin treatment device in accordance with embodiments of the present invention; and
Fig. 4 shows a transmission for moving a mechanical finger of a skin treatment device, in accordance with an embodiment of the present invention.

### DETAILED DESCRIPTION OF EMBODIMENTS

In the following detailed description, numerous specific details are set forth in order to provide a thorough understanding of embodiments of the invention. However, it will be understood by those of ordinary skill in the art that the invention may be practiced without these specific details. In other instances, well-known methods, procedures, components, modules, units and/or circuits have not been described in detail so as not to obscure the invention.

In accordance with embodiments of the present invention, a skin treatment device includes two or more radiofrequency (RF) electrodes. Each radiofrequency electrode is incorporated in a mechanical finger. One or more of the mechanical fingers is movable with a back-and-forth motion.

The skin treatment device may include a mechanism for coordinated movement of two or more of the mechanical fingers. For example, the coordinated movement may enable distal ends of two mechanical fingers to alternately approach and recede from one another. The motion may be repetitive or periodic.

A frequency or speed of the motion of a mechanical finger may be adjustable. Similarly, a distance through which a mechanical finger is moved during the course of the motion (e.g. amplitude of the motion) may be adjustable.
Alternatively to coordinated motion of two or more mechanical fingers, one mechanical finger may be moved relative to one or more stationary neighboring mechanical fingers. Reference herein to motion of mechanical fingers should be understood as referring to motion of the mechanical fingers relative to one another, regardless of how many of the mechanical fingers are moved relative to the remainder of the skin treatment device.

The skin treatment device includes electronic circuitry for applying a radiofrequency electromagnetic signal to a radiofrequency electrode that is incorporated into each mechanical finger. For example, radiofrequency electromagnetic signals of opposite polarity may be applied concurrently to two (or more) of the mechanical fingers. A frequency or amplitude (and thus a power) of the radiofrequency signal may be adjustable. The frequency, amplitude, or another characteristic of the radiofrequency signal may be generated so as to change in a periodic manner. For example, a variation of a characteristic of a radiofrequency signal that is applied to a mechanical finger may be coordinated with motion of the mechanical finger.

A skin treatment device in accordance with embodiments of the present invention may be portable. For example, components of the skin treatment device may be contained within a single housing. The housing may be of such a size as to enable the skin treatment device to be held in one hand. The skin treatment device may be shaped so as to be securely and comfortably held in the hand.

A skin treatment device in accordance with embodiments of the present invention may be placed in contact with skin and operated. For example, a user of the device may hold the skin treatment device such that the mechanical fingers are placed in contact with the user's own skin, or with the skin of another person whose skin is being treated by the user. Operation of the skin treatment device may include moving the mechanical fingers while concurrently applying a radiofrequency electromagnetic signal to the mechanical fingers.

Mechanical movement of the mechanical fingers may massage the skin to which the skin treatment device is applied. For example, the mechanical fingers may be move in a periodic back-and-forth motion toward and away from each other. The periodic back-and-forth motion may then alternately squeeze (pinch) and stretch the skin that is between the mechanical fingers. Movement of the mechanical fingers may be configured so as to assure that the resulting massaging is enjoyable or tolerable, without causing any pain or discomfort (e.g. by applying an excessive force to the skin). The user may manually adjust the force with which the skin treatment device is pressed against the skin being treated.

Concurrent application of the radiofrequency electromagnetic signal to the skin may cause a radiofrequency electrical current to flow through the skin. The flow of radiofrequency electrical current through the skin may then heat the skin (electro-thermolysis of the skin).

The depth at which a radiofrequency electrical current flows below the outer surface of the skin may be determined by one or more factors. The factors may include, for example, the frequency of the applied radiofrequency electromagnetic signal, the temperature of the skin, the conductivity of the skin, and a separation distance between the electrodes (each electrode at a distal end of one of the mechanical fingers, or being identical with an outer surface of a mechanical finger) through which the radiofrequency electromagnetic signal is applied to the skin. A motion of the mechanical fingers in which the distal ends of two mechanical fingers alternately approach and retreat from one another causes the separation distance between the electrodes to increase and decrease during the motion. The increase and decrease of the separation distance may thus vary the depth of radiofrequency electrical current, and thus of heating (electro-thermolysis), within the skin. Thus, the application of a radiofrequency electromagnetic signal to the skin concurrently with a back-and-forth motion of the electrodes may enable repetitive heating of layers within the skin at a range of depths.

Control of the frequency of the applied radiofrequency electromagnetic signal during the course of the back-and-forth motion of the mechanical fingers may enable further control of the depth of the heating. For example, the frequency of the applied radiofrequency electromagnetic signal may be coordinated with the back-and-forth motion of the mechanical fingers such that radiofrequency electrical current flows at an approximately constant depth. In this case, the skin may be heated at an approximately constant depth throughout the back-and-forth motion of the mechanical fingers. As another example, the frequency of the applied radiofrequency electromagnetic signal may be coordinated with the back-and-forth motion of the mechanical fingers so as to increase the range of depths through which the radiofrequency electrical current flows. Thus, a range of depths at which the skin is heated may be further increased.

One or more of the mechanical fingers may be provided with a temperature sensor. The temperature sensor may be placed in thermal contact with the skin when the skin treatment device or the mechanical fingers of the skin treatment device are placed in contact with the skin. The temperature sensor may generate a voltage or other electrical signal that is indicative of a measured temperature of the skin.

Circuitry or a controller for controlling generation of the radiofrequency electromagnetic signal may be coupled to the temperature sensor. The circuitry may be configured such that if a signal that is generated by the temperature sensor indicates that the skin has been warmed beyond a predetermined threshold value, generation of the radiofrequency electromagnetic signal is interrupted (e.g. stopped or continued with smaller amplitude).

A skin treatment device in accordance with embodiments of the present invention may be configured for home use or for use by a nonprofessional user. For example, the skin treatment device may be sufficiently small so to enable storage of the skin treatment device in a similar manner to other home cosmetic or beauty products and devices. Also, a small size and an ergonomic shape of the skin treatment device may enable the skin treatment device to be handled and operated without the assistance of any fixtures or other support structure. The skin treatment device may include built-in safety features that enable safe operation by a nonprofessional user.

Treatment of skin using a skin treatment device in accordance with embodiments of the present invention may be advantageous. A skin treatment device as described herein may be continually slid or glided over the skin surface concurrently with operation of the skin treatment device (e.g. while concurrently applying a radiofrequency electromagnetic signal to the skin and massaging the skin). On the other hand, some previously described skin treatment devices utilize a vacuum to attach to or grab a region of skin to be treated. With such a previously described device, it would be necessary to break the vacuum before moving the device to another region of the skin. Thus, it would not be possible to move such a previously described device over skin during use.

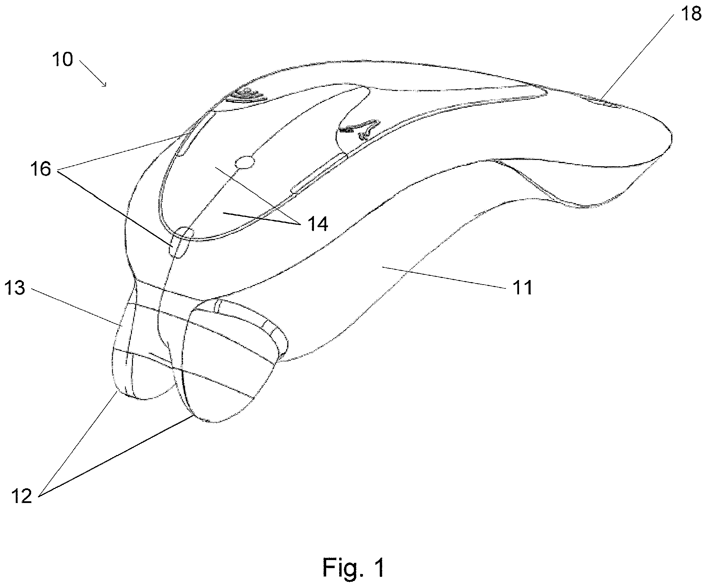

Fig. 1 shows a skin treatment device in accordance with an embodiment of the present invention.

Skin treatment device 10 is enclosed within housing 11. Housing 11 may configured with an ergonomic shape so as to enable skin treatment device 10 to be held in a single hand by a user. The shape of housing 11 may be configured so as to enable holding or pressing mechanical fingers 12 against the skin to be treated. The shape of housing 11 may also enable convenient and effective manipulation of skin treatment device 10. Housing 11 may include a rigid electrically insulating material (e.g. plastic) so as to electrically and mechanically isolate a user of skin treatment device 10 from any internal electrical or mechanical components of skin treatment device 10. Housing 11 may be designed to prevent isolate internal components of skin treatment device 10 from environmental factors (e.g. moisture or corrosive materials) that may adversely affect operation of the internal components.

Mechanical fingers 12 extend from an end of skin treatment device 10 that is configured to be placed against the skin to be treated. Mechanical fingers 12 are configured to move in a coordinated motion alternately toward and away from one another. Thus, when placed against skin, the coordinated motion of mechanical fingers 12 may result in a gentle pinching and releasing of a region of skin between mechanical fingers 12. Thus, the skin may be massaged by the motion of mechanical fingers 12.

Each mechanical finger 12 may be contoured (e.g. without sharp edges or corners) so as to prevent scratching of the skin or to slip over the skin. A surface of the mechanical finger 12 may be ridged, embossed, pitted, or otherwise patterned so as to facilitate grabbing or gripping the skin.

A material for an outer surface of each mechanical finger 12 may be selected so as to avoid irritation or chafing of the skin. Prior to or during use, a lubricating (e.g. an electrically conducting) fluid, gel, or cream may be applied to each mechanical finger 12.

Band 13 of housing 11 may be made of a flexible material, such as flexible plastic or rubber. The flexibility of band 13 may enable housing 11 to remain sealed while enabling motion of mechanical fingers 12.

In the illustrated embodiment, skin treatment device 10 includes two mechanical fingers 12. In other embodiments, more than two mechanical fingers may be included. For example, a plurality of mechanical fingers may be arranged in cooperating pairs. The mechanical fingers of each cooperating pair may move in a coordinated motion. In other embodiments, three or more mechanical fingers may be placed in a single row and caused to move with a coordinated motion. For example, a mechanical finger that is located between two neighboring mechanical fingers may alternately move toward one of the neighboring mechanical fingers and then toward the other. The neighboring mechanical fingers may be stationary or moveable.

Part or all of an outer surface of each mechanical finger 12 may include, or may be made of, a conducting material. For example, the conducting material may include a metal, a conducting plastic, or another conducting material suitable for inclusion in an outer surface of mechanical finger 12. Inclusion of the conducting material may facilitate electrical coupling of the mechanical finger 12 to the skin to be treated.

Skin treatment device 10 may include one or more controls 14. For example, controls may include one or more pushbuttons (as shown), switches, levers, dial wheels, or knobs. A user may operate a control 14 so as to control operation of skin treatment device 10. For example, operation of a control 14 may cause skin treatment device 10 to be powered on or off, or to enter a standby status. Operation of control 14 may indicate selection of a mode of operation of skin treatment device 10.

Operation of a control 14 may start or stop motion of mechanical fingers 12. Operation of a control 14 may change or select a speed, distance (e.g. amplitude) or other characteristic of the motion. For example, operation of a control 14 in the form of a pushbutton may change a state of motion of mechanical fingers 12 from one state to a successive state in accordance with a predetermined sequence of states. As another example, control 14 may be operated to select a characteristic of the motion from among a set of predetermined characteristics.

Operation of a control 14 may initiate or stop generation of a radiofrequency electromagnetic signal for conduction into the skin by mechanical fingers 12. Operation of a control 14 may cause resumption of generation of radiofrequency electromagnetic signal after an interruption, e.g. as caused in response to a detected high skin temperature. Operation of a control 14 may change a frequency, amplitude, or other characteristic of a radiofrequency electromagnetic signal that is applied to mechanical fingers 12. For example, successive operation of a control 14 may cause one or more characteristics of the radiofrequency electromagnetic signal to change in accordance with a predetermined sequence of signal characteristics.

Skin treatment device 10 may include one or more indicators 16. For example, an indicator 16 may include a light-emitting or other visible indication of a status of skin treatment device 10. Indicator 16 may include a light emitting diode (LED), light bulb, or other light generating device placed on or near the surface of skin treatment device 10 or housing 11 (e.g. protected by or enclosed in a transparent or translucent window, dome, or casing). Indicator 16 may include a light emitting device (e.g. light bulb or LED) that is located interior to skin treatment device 10, with its light being channeled to an outer surface of skin treatment device 10 or housing 11 (e.g. by an optical fiber or other light guide). For example, one or more indicators 16 may indicate a power state of skin treatment device 10 (e.g. power on, power off, standby).

One or more indicators 16 may indicate a current motion state of mechanical fingers 12. A motion state may include, for example, the motion being on or off, a speed, amplitude, or other characteristic of motion, or a currently selected motion state from among a limited set of predetermined motion states.

One or more indicators 16 may indicate a current state of a radiofrequency electromagnetic signal motion that is currently applied by mechanical fingers 12. A state of an applied radiofrequency electromagnetic signal may include, for example, whether signal generation is currently turned on or off, a frequency, amplitude, or other characteristic of the signal, or whether current generation has been interrupted (e.g. due to detected high skin temperature).

In addition to, or in place of, a visible indication, skin treatment device 10 may configured to generate an audible signal to indicate a current state of, or a change in a current state of, skin treatment device 10.

Skin treatment device 10 may include one or more connectors 18. A connector 18 may be configured to connect to a corresponding connector of an appropriate cable.

For example, a connector 18 may be configured to enable connection of skin treatment device 10 to an external power source (e.g. electric mains or power grid, or a power adapter, converter, or transformer) via an appropriate power cable. In accordance with some embodiments of a skin treatment device 10, skin treatment device 10 may incorporate an internal power source. For example an internal power source may include a replaceable of rechargeable battery or cell. A rechargeable battery may be recharged by connecting an appropriate connector 18 to an external power source. In other embodiments, power to operate skin treatment device 10 is provided solely by an external power source that is connected to a connector 18.

A connector 18 may be configured to connect an incorporated processor of skin treatment device 10 to a data port of an external computer, controller, or other device. Such a data connection may enable, for example, configuration of skin treatment device 10. In such a case, parameters for operation of skin treatment device 10 may be set or modified by a user or external device. In this manner, a skin treatment device 10 may be configured in accordance with, e.g., preferences of a user, or in accordance with changing manufacturer's recommendations (e.g. particular motion protocols for motion of mechanical fingers 12, or for generation of particular radiofrequency electromagnetic signals). Such a data connection may also facilitate diagnosis of problems with operation of a skin treatment device 10.

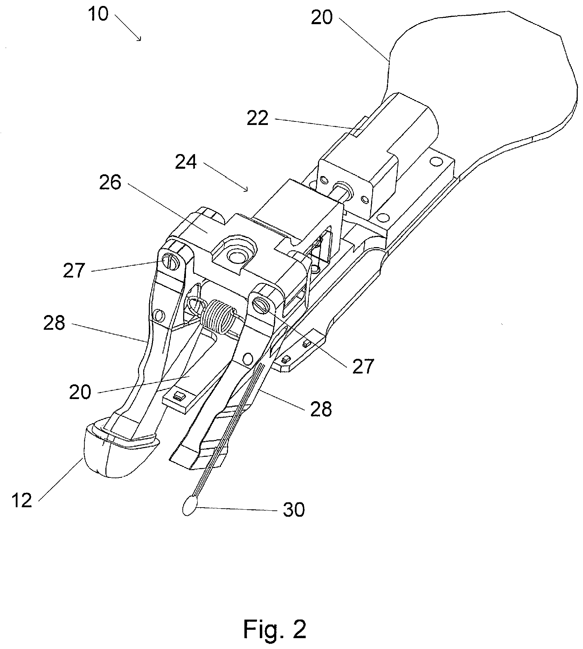

Fig. 2 shows internal components of the skin treatment device shown in Fig. 1. In Fig. 2, some of the components are shown schematically.

The electrical system 20 of skin treatment device 10 is represented schematically by a printed circuit board. Electrical system 20 may be considered to represent all internal electrical and electronic circuitry and components of skin treatment device 10.

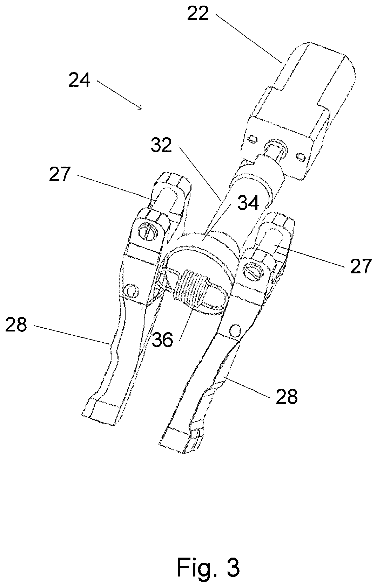

Fig. 3 is a block diagram of an electrical system for operation of a skin treatment device in accordance with embodiments of the present invention.

Electrical system 20 may include a controller 40 for controlling operation of skin treatment device 10. For example, the controller may include a controller 40. Controller 40 may represent a unit, or a plurality of intercommunicating units, that is configured to coordinate among various components of electrical system 20. Controller 40 may include one or more processors that are configured to operate in accordance with programmed instructions. In another example, controller 40 may include circuitry (e.g. in the form of an integrated control circuit) that is configured to operate components of skin treatment device 10.

Controller 40 may communicate with a data storage unit 42. Data storage unit 42 may include one or more volatile or non-volatile data storage devices, or may represent a data storage function of a device. For example, data storage unit 42 may include a non-volatile data storage device for storing programmed instructions or data for operation of a skin treatment device in accordance with some embodiments of the present invention. Data storage unit 42 may be used to store data that is generated during operation of the skin treatment device by a controller 40 in the form of processor.

Controller 40 may be configured to detect operation of a control 14. Controller 40 may operate in accordance with a detected operation of one or more controls 14 by a user.

Controller 40 may be configured to operate an indicator 16. For example, an indicator 16 may be operated to indicate a current state or status of operation of controller 40 or a component of electrical system 20.

Controller 40 may communicate with components and circuitry of generating a radiofrequency electromagnetic signal. Radiofrequency (RF) controller 44 may control generation of a radiofrequency electromagnetic signal. Radiofrequency controller 44 may represent one or more components or modules of controller 40, may include components that are separate from controller 40, or both. Radiofrequency controller 44 may be configured to select a radiofrequency electromagnetic signal to be generated by radiofrequency generator 46. A radiofrequency electromagnetic signal that is generated by radiofrequency generator 46 may be conducted to one or more mechanical arms 28, or to a conducting element that is incorporated into a mechanical arm 28. Mechanical arms 28 may conduct the generated radiofrequency electromagnetic signal to each mechanical finger 12. The generated radiofrequency electromagnetic signal may induce a radiofrequency electrical current to flow in skin that is in contact with mechanical fingers 12.

Radiofrequency controller 44 may select a radiofrequency electromagnetic signal on the basis of an indication based on operation by a user of a control 14. Generation of a radiofrequency electromagnetic signal may be controlled in accordance with a skin temperature that is sensed by temperature sensor 30. For example, if a sensed temperature is above a threshold temperature value, radiofrequency controller 44 may stop generation of a radiofrequency electromagnetic signal by radiofrequency generator 46.

Controller 40 may communicate with components and circuitry for controlling motion of moving components, such as mechanical fingers 12. Motion controller 48 may be configured to operate motor 22. For example, motion controller 48 may be configured to adjust an electrical current that is provided to drive motor 22 so as to operate drive motor 22 at a particular speed. Operation of drive motor 22 may cause mechanical fingers 12 to move in a predetermined manner.

Motion controller 48 may be configured to monitor an electrical current that is supplied to drive motor 22. For example, monitoring a current that is supplied to drive motor 22 may enable detecting a state where motion of a mechanical arm 28 or a mechanical finger 12 is impeded or obstructed.

Controller 40 may be configured to operate motion controller 48 in coordination with radiofrequency controller 44. For example, operation of motion controller 48 may be coordinated with operation of radiofrequency controller 44 so as to generate a radiofrequency electromagnetic signal that depends on a current configuration of (e.g. separation distance between) mechanical fingers 12. For example, drive motor 22 or one or more components of transmission 24 may be provided with an encoder or other sensing device for sensing a current orientation or position of a mechanical arm 28 (e.g. derived from an sensed orientation of a cam 34). Coordination of operation of motion controller 48 with radiofrequency controller 44 may enable control of a depth at which a radiofrequency electrical current flows through the skin or underlying tissue.

Electrical system 20 may include a power supply 50. Power supply 50 may provide power for operation of controller 40 or for operation of another component of electrical system 20. Power supply 50 may include a replaceable or rechargeable power source, such as a replaceable or rechargeable battery or cell. Power supply 50 may connect to an external power source, such as an electric power grid, generator, or power supply device, via connector 18. Power supply 50 may include an appropriate converter or transformer for converting an input electrical current from connector 18 to a current that is usable in operating one or more components of the skin treatment device.

Electrical system 20 may connect to or communicate with one or more temperature sensors 30. A temperature sensor 30 may be mounted on a mechanical arm 28. Temperature sensor 30 may be embedded within, or may be in thermal contact with, a mechanical finger 12 that is placed at a distal end of mechanical arm 28.

Operation of drive motor 22 may cause motion of a mechanical finger 12. For example, a rotational motion of drive motor 22 may be converted by a transmission 24 to a back-and-forth motion of one or more mechanical arms 28. A mechanical arm 28 may be mounted to arm base 26 via an axis 27. Mechanical arm 28 may thus rotate about axis 27 relative to arm base 26. Thus, operation of drive motor 22 and transmission 24 may cause a mechanical finger 12 at a distal end of a mechanical arm 28 to rotate with a back-and-forth motion about axis 27.

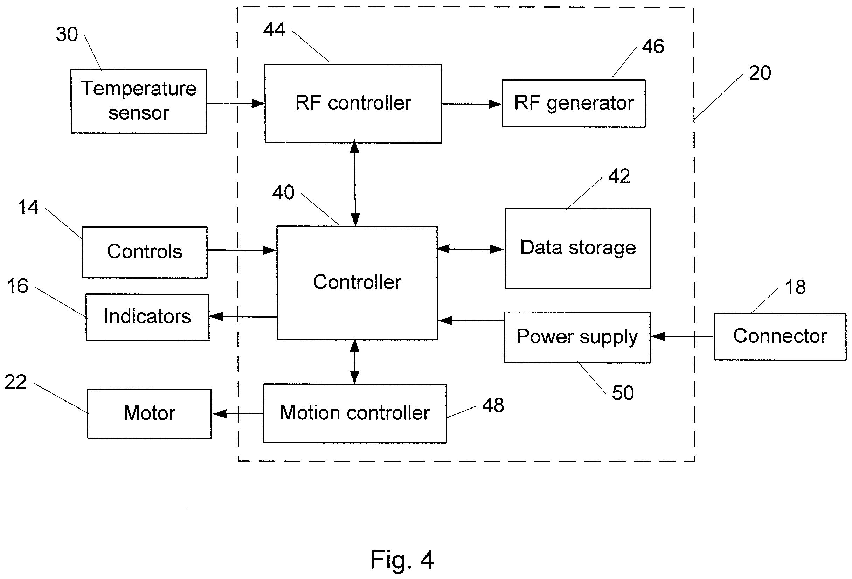

Fig. 4 shows a transmission for moving a mechanical finger of a skin treatment device, in accordance with an embodiment of the present invention.

Drive motor 22 is coupled to drive shaft 32 of transmission 24. Thus, a torque that is produced by operation of drive motor 22 may rotate drive shaft 32. An end of drive shaft 32 that is distal to drive motor 22 is coupled to cam 34 of transmission 24. Thus, rotation of drive shaft 32 causes cam 34 to rotate.

Mechanical arms 28 are mounted to arm base 26 (shown in Fig. 2) via axes 27. Thus, a mechanical arm 28 may be made to rotate about its axis 27. Mechanical arms 28 are connected to one another by spring 36. Spring 36 may provide an inward restoring force or torque when one mechanical arm 28 is rotated away from the other.

Cam 34 is placed between mechanical arms 28. Cam 34 thus counters the inward torque that is exerted by spring 26 so as to separate one mechanical arm 28 from the other. Cam 34 has an elliptical shape. Thus, rotation of cam 34 continually changes the distance of separation between one mechanical arm 28 and the other. For example, when cam 34 is rotated such that the major axis of the elliptical shape separates mechanical arms 28, the separation distance between mechanical fingers 12 (Fig. 1) from one another is close to its maximum. On the other hand, when cam 34 is rotated such that the minor axis of the elliptical shape separates mechanical arms 28, the separation distance between mechanical fingers 12 is close to its minimum.

Spring 26 may be selected or configured so as to limit a maximum inward force that may be exerted by spring 26 on mechanical arms 28. The force limit may be selected or configured such that the maximum force is too weak to cause any pain (such as painful pinching) or damage to any skin that may be positioned between mechanical fingers 12.

In the configuration shown in Figs. 2 and 4, the motion of both mechanical arms 28 and of both mechanical fingers 12 is symmetric and opposite. Other configurations in which the mechanical fingers move in an asymmetric manner are possible.

Although both mechanical arms in Fig. 4 are shown as rotatable about their respective axes 27, other configurations are possible. For example, one mechanical arm may be rotatable, while the other is stationary. As another example, each mechanical arm may be moved, but in a nonsymmetrical manner. For example, as an alternative to a single symmetrically mounted elliptically-shaped cam 34, each mechanical arm may be operated by a separate cam, or by a shared cam with an asymmetric shape, or by a cam with a symmetric shape that is mounted eccentrically on its axis of rotation. A restoring force may be separately provided to each mechanical arm. For example, a separate spring or piston connecting each mechanical arm to a stationary element of the skin treatment device may provide a separate restoring force to each of the mechanical arms.

Adjustment of a speed of operation of motor 22 may adjust a speed with which mechanical arms 28 and mechanical fingers 12 are moved.

A mechanism may be provided to adjust an amplitude or other distance of motion of a mechanical arm 28 or a mechanical finger 12. For example, a distance between axes 27 may be adjustable. A mechanism may be provided (e.g. manual or motorized) for changing a distance between axes 27.

In some embodiments of the present invention, a length of a mechanical arm 28 or a mechanical finger 12 may be adjustable. An attachment point of mechanical arm 28 to an axis 27, or of mechanical finger 12 to mechanical arm 28 may be adjustable (e.g. manually detachable and re-attachable). Mechanical arm 28 may be constructed with two or more telescoping sections so as to enable adjustment of the length of mechanical arm 28.

In some embodiments of the present invention, a transmission with a rotating cam may be configured such that lateral translation of the cam causes an amplitude of a motion of a mechanical arm to change. (A separate motor or transmission may be provided to effect or enable such motion.) For example, adjustment of a distance between an axis of rotation of the cam and the axes of the mechanical arms may adjust an amplitude of the motion of the mechanical arms. In another example, a cam may be in the form of a frustum of a cone (e.g. of an elliptical cone) or of a stepped cone. In this case, translating the cam along, or parallel to, the cams axis of rotation may cause the dimensions of the section of the cam that lies between the mechanical arms to change. Changing dimensions of the section of the cam between the mechanical arms may adjust the amplitude of the motion of the mechanical arms and of the attached mechanical fingers.

A skin treatment method in accordance with embodiments of the present invention may include operation of a skin treatment device in accordance with embodiments of the present invention.

In accordance with the treatment method, at least two mechanical fingers of the skin treatment device are placed in contact with skin that is to be treated. A motor of the skin treatment device may be operated so as to continuously move (to be understood as including a sequence of intermittent movements) at least one of the mechanical fingers relative to the other. As a result of the motion, a separation distance between the mechanical fingers is continuously changed. The relative motion of mechanical fingers may massage the skin.

Concurrently with the motion, a radiofrequency generator may be operated so as to generate a radiofrequency electromagnetic signal that is conducted to the two mechanical fingers. A radiofrequency electrical current may thus be conducted by the skin from one of the mechanical fingers to the other. The depth at which the current is conducted through the skin may be affected by the separation distance between the mechanical fingers. As a result of the current, the skin near the conduction depth may be heated.

Thus, if characteristics of the generated radiofrequency electromagnetic signal, the depth of the current may change as a result of the motion of the mechanical fingers. Alternatively, one or more characteristics (e.g. a frequency) of the generated radiofrequency electromagnetic signal may be changed in coordination with the motion of the mechanical fingers. Such coordinated generation of the radiofrequency electromagnetic signal may be applied so as to exert precise control over heating of layers of the skin at different depths.

## Claims

1. A device (10) for treating skin, the device (10) comprising:
a motor (22) and a transmission (24);
two mechanical fingers (12) for placement in contact with the skin, at least one of the mechanical fingers (12) being configured to be moved by the motor (22) and the transmission (24) in a coordinated motion alternately toward and away from one another so as to change a separation distance between the mechanical fingers (12) and to gently pinch and release a region of skin between the mechanical fingers (12) to massage the skin, the mechanical fingers (12) being configured to concurrently conduct a generated radiofrequency electromagnetic signal to the skin so as to cause an electrical current to flow through the skin at a depth that corresponds to the separation distance; and
a temperature sensor (30) for sensing a temperature of the skin, wherein the temperature sensor (30) is embedded in, or is in thermal contact with, one of the mechanical fingers (12).

2. A device (10) according to claim 1, wherein the device (10) is shaped so as to be held by a single hand.

3. A device (10) according to claim 2, wherein the transmission (24) comprises a cam (34) that is rotatable by the motor (22) for changing a distance between the fingers (12).

4. A device (10) according to claim 3, wherein each of the fingers (12) is located at a distal end of an arm (28) that is rotatable about an axis (27), and wherein the cam (34) is located so as to contact the arm (28) at a point between the distal end of the arm (28) and the axis (27) of the arm (28).

5. A device (10) according to claim 4, comprising a spring (36) for exerting an inward torque on at least one of the arms (28).

6. A device (10) according to claim 1, comprising a controller (40) for controlling operation of the motor (22).

7. A device (10) according to any of the preceding claims, wherein the radiofrequency electromagnetic signal is generated in accordance with the sensed temperature.

8. A device (10) according to any of the preceding claims, wherein the device (10) is configured to interrupt generation of the radiofrequency electromagnetic signal if the sensed temperature exceeds a threshold temperature.

9. A device (10) according to any of the preceding claims, comprising a control (14) for operation by a user.

10. A device (10) according to any of the preceding claims, comprising an indicator (16) for indicating a status of the device (10).

11. A device (10) according to any of the preceding claims, comprising a power supply (50).

12. A device (10) according to claim 11, wherein the power supply (50) comprises a rechargeable battery or a replaceable battery.

## Patentansprüche

1. Vorrichtung (10) zur Behandlung von Haut, wobei die Vorrichtung (10) umfasst:
einen Motor (22) und ein Getriebe (24);
zwei mechanische Finger (12), die mit der Haut in Kontakt zu bringen sind, wobei mindestens einer der mechanischen Finger (12) so eingerichtet ist, durch den Motor (22) und das Getriebe (24) in einer koordinierten Bewegung abwechselnd zu einander hin und von einander weg bewegt zu werden, so dass ein Trennungsabstand zwischen den mechanischen Fingern (12) verändert wird und dass eine Hautregion zwischen den mechanischen Fingern (12) sanft zusammengedrückt und freigegeben wird, so dass die Haut massiert wird,
wobei die mechanischen Finger (12) so eingerichtet sind, dass sie gleichzeitig ein generiertes elektromagnetisches Hochfrequenzsignal zu der Haut hin leiten, so dass bewirkt wird, dass ein elektrischer Strom in einer Tiefe durch die Haut fließt, welche dem Trennungsabstand entspricht; und
ein Temperatursensor (30) zum Abtasten einer Temperatur der Haut, wobei der Temperatursensor (30) in einem der mechanischen Finger (12) eingebettet ist oder in Thermokontakt mit einem der mechanischen Finger (12) steht.

2. Vorrichtung (10) gemäß Anspruch 1, wobei die Vorrichtung (10) so geformt ist, dass sie in einer einzigen Hand gehalten werden kann.

3. Vorrichtung (10) gemäß Anspruch 2, wobei das Getriebe (24) einen Nocken (34) umfasst, welcher sich durch den Motor (22) drehen lässt, so dass der Abstand zwischen den Fingern (12) verändert wird.

4. Vorrichtung (10) gemäß Anspruch 3, wobei sich jeder der Finger (12) an einem distalen Ende eines Arms (28) befindet, welches sich um eine Achse (27) drehen lässt, und wobei der Nocken (34) so platziert ist, dass er mit dem Arm (28) an einem Punkt zwischen dem distalen Ende des Arms (28) und der Achse (27) des Arms (28) in Kontakt kommt.

5. Vorrichtung (10) gemäß Anspruch 4, eine Feder (36) zum Ausüben eines einwärts gerichteten Drehmoments auf mindestens einen der Arme (28) umfassend.

6. Vorrichtung (10) gemäß Anspruch 1, einen Controller (40) zum Steuern bzw. Regeln des Betriebs des Motors (22) umfassend.

7. Vorrichtung (10) gemäß einem beliebigen der vorangehenden Ansprüche, wobei das elektromagnetische Hochfrequenzsignal in Übereinstimmung mit der abgetasteten Temperatur erzeugt wird.

8. Vorrichtung (10) gemäß einem beliebigen der vorangehenden Ansprüche, wobei die Vorrichtung (10) so eingerichtet ist, dass sie die Generierung des elektromagnetischen Hochfrequenzsignals unterbricht, wenn die abgetastete Temperatur eine Schwellentemperatur übersteigt.

9. Vorrichtung (10) gemäß einem beliebigen der vorangehenden Ansprüche, einen Controller (14) zur Bedienung durch einen Benutzer umfassend.

10. Vorrichtung (10) gemäß einem beliebigen der vorangehenden Ansprüche, ein Anzeigegerät (16) zum Anzeigen eines Zustandes der Vorrichtung (10) umfassend.

11. Vorrichtung (10) gemäß einem beliebigen der vorangehenden Ansprüche, eine Stromversorgung (50) umfassend.

12. Vorrichtung (10) gemäß Anspruch 11, wobei die Stromversorgung (50) eine wiederaufladbare Batterie oder eine auswechselbare Batterie umfasst.

## Revendications

1. Dispositif (10) de traitement de la peau, le dispositif (10) comprenant :
un moteur (22) et une transmission (24) ;
deux doigts mécaniques (12) destinés à être mis en contact avec la peau, au moins l'un des doigts mécaniques (12) étant configuré pour être déplacé par le moteur (22) et la transmission (24) selon un mouvement coordonné se rapprochant et s'éloignant alternativement l'un de l'autre pour modifier une distance de séparation entre les doigts mécaniques (12) et pour doucement pincer et libérer une région de peau entre les doigts mécaniques (12) pour masser la peau, les doigts mécaniques (12) étant configurés pour conduire simultanément un signal électromagnétique radiofréquence généré vers la peau afin de faire passer un courant électrique à travers la peau à une profondeur qui correspond à la distance de séparation ; et
un capteur de température (30) destiné à détecter une température de la peau, dans lequel le capteur de température (30) est intégré dans, ou est en contact thermique avec, l'un des doigts mécaniques (12).

2. Dispositif (10) selon la revendication 1, dans lequel le dispositif (10) a une forme lui permettant d'être maintenu par une seule main.

3. Dispositif (10) selon la revendication 2, dans lequel la transmission (24) comprend une came (34) qui peut être entraînée en rotation par le moteur (22) pour modifier une distance entre les doigts (12).

4. Dispositif (10) selon la revendication 3, dans lequel chacun des doigts (12) est situé au niveau d'une extrémité distale d'un bras (28) qui peut pivoter autour d'un axe (27), et dans lequel la came (34) est située de manière à entrer en contact avec le bras (28) au niveau d'un point entre l'extrémité distale du bras (28) et l'axe (27) du bras (28).

5. Dispositif (10) selon la revendication 4, comprenant un ressort (36) destiné à exercer un couple vers l'intérieur sur au moins l'un des bras (28).

6. Dispositif (10) selon la revendication 1, comprenant un organe de commande (40) destiné à commander l'actionnement du moteur (22).

7. Dispositif (10) selon l'une quelconque des revendications précédentes, dans lequel le signal électromagnétique radiofréquence est généré en fonction de la température détectée.

8. Dispositif (10) selon l'une quelconque des revendications précédentes, dans lequel le dispositif (10) est configuré pour interrompre la génération d'un signal électromagnétique radiofréquence si la température détectée excède une température seuil.

9. Dispositif (10) selon l'une quelconque des revendications précédentes, comprenant une commande (14) destinée à être actionnée par un utilisateur.

10. Dispositif (10) selon l'une quelconque des revendications précédentes, comprenant un indicateur (16) destiné à indiquer un statut du dispositif (10).

11. Dispositif (10) selon l'une quelconque des revendications précédentes, comprenant une source d'énergie (50).

12. Dispositif (10) selon la revendication 11, dans lequel la source d'énergie (50) comprend une batterie rechargeable ou une batterie jetable.
